# EUROPEAN PATENT SPECIFICATION

(11) **EP 0 650 365 B1**
(45) Date of publication and mention of the grant of the patent: **14.05.1997**
(21) Application number: 93914893.8
(22) Date of filing: 06.07.1993
(51) Int. Cl.: A61K 35/78, A61K 33/24, A61K 31/70

(54) **BIOCIDAL COMPOSITION CONTAINING MUSTARD SEED PLANT EXTRACT**
BIOZIDE ZUSAMMENSETZUNG DIE SENFSAMENEXTRAKT ENTHÄLT
COMPOSITION BIOCIDE CONTENANT DE L'EXTRAIT DE PLANTE DE MOUTARDE

(30) Priority: 07.07.1992 GB 9214419
(43) Date of publication of application: 03.05.1995
(73) Proprietor: Alatese Ltd, Kingson-upon-Hull HU1 1XE (GB)
(72) Inventor: Concannon, Peter, Malton, North Yorkshire YO17 8JW (GB)
(74) Representative: Harrison, Michael Robert
(86) International application number: GB9301417
(87) International publication number: WO9401121

(56) References cited:
- DE-A- 2 046 756
- GB-A- 2 107 583
- US-A- 4 851 398
- BIOLOGICAL ABSTRACTS, vol. 87, no. 9, Philadelphia, PA, US; abstract no. 97201; SPAK J.: 'The effect of the glucosinolate sinigrin and of allyl isothiocyanate on the infectivity of turnip mosaic virus', page 881-882

## Description

The invention relates to a biocidal agent and in particular to an agent which is antibacterial, antifungal and antiviral. To date, the agent of the invention is being used to treat a wide variety of diseases in animals and humans - both internally and externally, in a localised or systemic manner. However, it is thought that the invention has particular application in the treatment of viruses and particularly, but not exclusively, equine viruses. This is mainly because there are few effective anti-viral agents on the market especially the equine market and there is therefore a need to provide a suitable anti-viral product.

The advantages of providing a suitable equine anti-viral product are apparent when one considers that a racehorse can cost millions of pounds and once infected with a virus it is temporarily unable to compete and thus bring about any return for money invested in the horse. At worst, viral infections can result in a fatality.

It is also of note that the agent in accordance with the invention is suitable for treating human viral conditions such as Herpes.

Further, the agent in accordance with the invention has also been used for treating a wide variety of conditions of a non-viral nature, for example, cuts, ulcers or burns.

The agent of the invention is a natural remedy in that it comprises the combination of known and harmless products, which products have either been consumed by or used by man for centuries. It was found that a combination of these products produced an agent which had unexpected anti-viral properties. Further, the agent also had unexpected efficacy when treating non-viral ailments. In this latter instance, it is thought that the selection of the components of the agent produced an agent having enhanced or synergistic effects thus providing a single agent which is particularly successful for treating non-viral ailments.

The agent in accordance with the invention is a biocidal agent which comprises a mixture of an alkaline substance and a botanical extract from the Cruciferous herbs commonly known as mustard seed producing plants.

In an ideal form of the invention the agent further comprises a sweetening substance which ideally is honey.

The agent of the invention thus comprises at least two and ideally three components. Each of these components are known to man and indeed are known to have beneficial medicinal properties. These agents and their properties will now be reviewed.

The preferred alkaline substance of the invention is a bismuth salt and preferably bismuth carbonate, ideally in its purest form such as bismuth carbonate BP pure grade.

Bismuth is one of the heavy metals whose carbonate, oxide, subnitrate, salicylate and oxychloride are essential ingredients in many pharmaceutical compounds. Salts of bismuth are reputed to form a protective covering over any ulcerated areas. The carbonate, subcarbonate, subnitrate, salicylate and the subgallate are still employed for the treatment of ulcers and other gastro-intestinal disorders, such as diarrhoea and vomiting. As astringents they are sometimes used for weeping sores on the surface of the skin, often mixed with starch and oxide of zinc.

Bismuth belongs to the metals which form only basic carbonates. (BiO)₂CO₃H₂O is a substance with a weak antacid effect reacting slowly with the hydrochloric acid of the stomach and inhibiting the secretion by coating the mucous membrane. Like the carbonates of magnesium and calcium it is employed in gastric or duodenal ulcer, also in cases of diarrhoea. Incorporated in ointments and dusting powders it has a protective and sedative effect on inflamed skin.

There is no record disclosing the use of bismuth and in particular bismuth carbonate to treat viral diseases.

The second agent of the invention is an extract from the Cruciferous herbs commonly known as mustard seed producing plants.

The pulverised ripe seeds of Cruciferous herbs yield a yellowish powder called mustard which is used for the condiment mustard or medicinal purposes. Further, the leaves are sometimes used as greens or pot herbs.

In medicine, mustard plasters which contain pulverised mustard seeds mixed with varying amounts of flour (to control the irritating qualities) and warm water (to make a smooth paste) have long been used as an irritant to the skin, to produce a local superficial inflammation of transitory nature. Internally, it is sometimes used as an emetic for dogs when other and more suitable substances are not to hand.

A salient characteristic of the mustard plants is that they contain a particular glycoside. The term glycoside is applied to a large number of substances found mainly in plants and result from secondary metabolism; usually they have a bitter taste. Their exact biological function is not established but it is probable that their formation provides the plant with a means of storing, in a harmless form, toxic and physiologically active materials which may be liberated by enzymes, when required. Most of the natural glycosides have their names from the botanical sources.

A glycoside is formed when sugars combine or react with non-carbohydrates. While compounds consisting of sugars only, are generally referred to as disaccharides, trisaccharides, etc, the word glycoside is restricted to compounds in which one or more sugars (the glycone component) are combined with a non-sugar (the aglycon). The aglycons are widely diversified in chemical structure but all contain a hydroxyl (OH) group through which they combine with the sugar.

When hydrolysed (split by the action of aqueous solutions of acids or enzymes) the glycosides separate into the glycone and aglycone components. Each glycoside usually has a compatible enzyme which, however, is situated in cells elsewhere in the plant. Crushing or ingesting the plant part breaks up the cells, the enzyme is brought into contact with the glycoside, hydrolysis occurs and the aglycone is activated.

Glycosides include some of the most effective plant drugs, and some of the plants containing them are among the most toxic known. The therapeutically active constituent is the aglycone, which can selectively affect a particular organ in the human body. As a rule the sugars have no therapeutic effect but they increase the solubility of the glycoside and its absorption in the body and can facilitate its transport to a specific organ.

Glucosinolates are glycosides with the same general formula (including that the sulphur-linked sugar is always glucose) and are in the *in vivo* precursors of the mustard oils. Although themselves odourless and tasteless, glucosinolates are hydrolysed to pungently odoured and tasting compounds (isothiocyanates), such as when the plant is damaged or when mustard seeds are ground. Also during extraction or isolation the obnoxious flavours are obvious.

About 75 glucosinolates of known structure have been isolated from higher plants, the majority being aliphatic (open-chain) derivatives (eg sinigrin) with the remainder having benzyl substitutes (eg sinapine) of one sort or another. The Cruciferae family is particularly rich in these compounds, especially the flowers and seeds.

The glucosinolates are always accompanied by the appropriate hydrolytic enzyme, a thioglucosidase known as myronsinase present in certain cells.

When the enzyme is activated, it catalyses the hydrolysis of the glycoside into its sugar component (glucose) and its aglycone (an allyl isothiocyanate - the mustard oil).

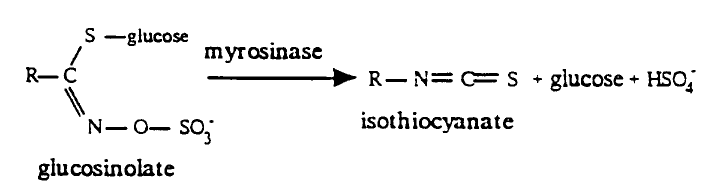

Mustard Oil (Ally Isothiocyanate) (or: essential/volatile oil of mustard) is a general term applied to the esters of isothiocyanic acid. Allyl isothiocyanates, C₃H₅.N:C:S, are colourless liquids sparingly soluble in water and boiling around 152°C. A few examples of glucosinolates are sinigrin, sinalbin and sinapine.

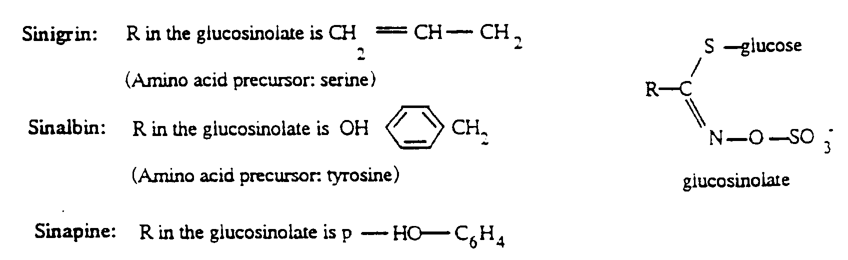

Other examples of glucosinolates include gluconapan, glucobrassicanapin, progoitrin, glucotropaeolin, gluconasturtin, glucobrassicin and four hydroxiglucobrassicin.

Isothiocyanates are antibacterial, antifungal and insecticidal and thus it would seem that the "purpose" of them is to protect the plant from invaders. The antiseptic properties are due to the activity of the sulphur compounds.

Mustard oil can be prepared synthetically by heating allyl bromide, ally chloride or allyl iodide with potassium thiocyanate.

When working the invention mustard seeds are ground together with bismuth and ideally honey to form a paste. It is not yet known what is the active substance from the mustard seeds but having regard to the above described chemistry it is highly likely that the active agent is the Aglycone component, that is to say the isothiocyanate and, more specifically, allyl isothiocyanate.

As mentioned above it is preferable to combine bismuth carbonate with mustard seeds and a sweetening agent such as honey.

Honey is a mixture of invert sugars and compound saccharides produced from the nectar of flowers by enzyme action in the honey sac of, typically, the bee *Apis mellifica*. Although there is well documented use of honey in medicine when mixed with various drugs as a soothing preparation, for example, for soothing a sore throat, there is no record of the use of honey for treating viral diseases, wounds, burns or the like.

It will be apparent that the invention encompasses not only an agent made of naturally occurring components but also an agent made of corresponding synthetic components.

An embodiment of the invention will now be described by way of example only.

The agent of the invention is manufactured by combining three components in the following proportions.

1 teaspoon of pure grade bismuth carbonate BP mixed with 2 tablespoons of mustard seeds, ideally *Sinapis-alba* or *Brassica juncea* and 1 tablespoon of sweetening agent such as honey.

The ingredients are intimately mixed using a pestle until a smooth paste is provided.

In a preferred embodiment of the invention, the two aforementioned mustard seeds are mixed together in equal amounts and then 2 tablespoons of the seeds are added to the 1 teaspoon of bismuth carbonate and 1 tablespoon of honey.

When the paste has been produced it can then be administered internally, by oral administration or externally by applying the paste to the area to be treated.

In *in vivo* experiments, the agent of the invention has proved successful in curing viral infections in horses, including Herpes viral infection.

Further, the agent has also been successful in curing Herpes viral infections in man and flu virus infections. Further the agent of the invention has proved useful in curing lesions, ulcers, skin ailments, burns and the like.

The efficacy and rapidity with which the agent of the invention acts to cure an ailment is striking and indeed characterises the agent of the invention.

Thus it can be seen that an agent in accordance with the invention provides an effective means of treating a wide variety of conditions in the human or animal whether internally, externally, localised or systemic.

## Claims

1. A biocidal agent comprising a mixture of an alkaline substance and mustard seeds.

2. A biocidal agent according to Claim 1 further comprising a sweetening agent.

3. A biocidal agent according to Claim 2 wherein the sweetening agent is honey.

4. A biocidal agent according to Claim 1 wherein the alkaline substance is bismuth carbonate.

5. A biocidal agent according to Claim 4 wherein the bismuth carbonate is pure grade.

6. A biocidal agent according to Claims 2 to 5 wherein the three components are mixed in the following ratios: one teaspoon of alkaline substance, two tablespoons of mustard seeds and one tablespoon of honey.

7. A biocidal agent according to Claim 6 wherein said components are pulverised theretogether so as to ensure intimate mixing.

8. A biocidal agent according to Claim 1 wherein the mustard seeds are *Sinapis-alba.*

9. A biocidal agent according to Claim 1 wherein the mustard seeds are *Brassica juncea.*

10. A biocidal agent according to Claim 1 wherein the mustard seeds are a mixture of *Sinapis-alba* or *Brassica juncea.*

11. A biocidal agent according to Claim 10 wherein equal amounts of said two seeds are provided.

12. A biocidal agent comprising a mixture of an alkaline substance, a botanical extract which extract is produced from the botanical family of plants commonly known as mustard seed producing plants and a sweetening substance.

13. A biocidal agent according to Claim 12 wherein the sweetening agent is honey.

14. A biocidal agent according to Claim 12 wherein the alkaline substance is bismuth carbonate.

15. A biocidal agent according to Claim 14 wherein the bismuth carbonate is pure grade.

16. A biocidal agent according to Claim 12 wherein the three components are mixed in the following ratios: one teaspoon of alkaline substance, two tablespoons of mustard seeds and one tablespoon of honey.

17. A biocidal agent according to Claim 12 wherein said components are pulverised theretogether so as to ensure intimate mixing.

18. A biocidal agent according to Claim 12 wherein the mustard seeds are *Finatif-alba.*

19. A biocidal agent according to Claim 12 wherein the mustard seeds are *Braffica juncea.*

20. A biocidal agent according to Claims 12 to 19 wherein said botanical extract is a glucoside.

21. A biocidal agent according to Claims 12 to 19 wherein said botanical extract is a glucosinolate.

22. A biocidal agent according to Claims 12 to 19 wherein said botanical extract is an isothiocyanate.

23. A biocidal agent according to Claims 12 to 19 wherein said botanical extract is sinigrin.

24. A biocidal agent according to Claims 12 to 19 wherein said botanical extract is sinalbin.

25. A biocidal agent according to Claims 12 to 19 wherein said botanical extract is sinapine.

26. A biocidal agent according to Claims 12 to 19 wherein said botanical extract is gluconapan.

27. A biocidal agent according to Claims 12 to 19 wherein said botanical extract is glucobrassicanipan.

28. A biocidal agent according to Claims 12 to 19 wherein said botanical extract is progoitrin.

29. A biocidal agent according to Claims 12 to 19 wherein said botanical extract is glucotropaeolin.

30. A biocidal agent according to Claims 12 to 19 wherein said botanical extract is gluconasturtin.

31. A biocidal agent according to Claims 12 to 19 wherein said botanical extract is glucobrassicin

32. A biocidal agent according to Claim 31 wherein said glucobrassicin is four-hydroxiglucobrassicin.

33. A biocidal agent according to Claims 12 to 32 wherein said extract comprises any combination of Claims 20 to 32.

## Patentansprüche

1. Biocides Mittel mit einer Mischung aus einer alkalischen Substanz und Senfsamen.

2. Biocides Mittel nach Anspruch 1, welches weiterhin Süßmittel umfaßt.

3. Biocides Mittel nach Anspruch 2, **dadurch gekennzeichnet**, daß das Süßmittel Honig ist.

4. Biocides Mittel nach Anspruch 1, **dadurch gekennzeichnet**, daß die alkalische Substanz Wismutkarbonat ist.

5. Biocides Mittel nach Anspruch 4, **dadurch gekennzeichnet**, daß das Wismutkarbonat reiner Qualität ist.

6. Biocides Mittel nach einem der Ansprüche 2 bis 5, **dadurch gekennzeichnet**, daß die drei Bestandteile in den folgenden Verhältnissen gemischt werden: ein Teelöffel alkalische Substanz, zwei Teelöffel Senfsamen und 1 Teelöffel Honig.

7. Biocides Mittel nach Anspruch 6, **dadurch gekennzeichnet**, daß die Bestandteile zusammen zerstäubt werden, um eine gute Durchmischung sicherzustellen.

8. Biocides Mittel nach Anspruch 1, **dadurch gekennzeichnet**, daß die Senfsamen Sinapis-alba-Samen sind.

9. Biocides Mittel nach Anspruch 1, **dadurch gekennzeichnet**, daß die Senfsamen Brassica-juncea-Samen sind.

10. Biocides Mittel nach Anspruch 1, **dadurch gekennzeichnet**, daß die Senfsamen eine Mischung aus Sinapis-alba und Brassica juncea-Samen sind.

11. Biocides Mittel nach Anspruch 10, **dadurch gekennzeichnet**, daß gleiche Mengen der beiden Samen verwendet werden.

12. Biocides Mittel, bestehend aus einer Mischung aus einer alkalischen Substanz, einem botanischen Extrakt, welches Extrakt aus einer botanischen Pflanzenfamilie, die allgemein als Senfsamen produzierende Pflanzen bekannt ist, hergestellt wird und einer süßenden Substanz.

13. Biocides Mittel nach Anspruch 12, **dadurch gekennzeichnet**, daß das Süßmittel Honig ist.

14. Biocides Mittel nach Anspruch 12, **dadurch gekennzeichnet**, daß die alkalische Substanz Wismutkarbonat ist.

15. Biocides Mittel nach Anspruch 14, **dadurch gekennzeichnet**, daß das Wismutkarbonat reiner Qualität ist.

16. Biocides Mittel nach Anspruch 12, **dadurch gekennzeichnet**, daß die drei Bestandteile im folgenden Verhältnis gemischt sind: ein Teelöffel alkalische Substanz, zwei Teelöffel Senfsamen und ein Teelöffel Honig.

17. Biocides Mittel nach Anspruch 12, **dadurch gekennzeichnet**, daß die genannten Bestandteile zusammen zerstäubt werden, um eine gute Mischung sicherzustellen.

18. Biocides Mittel nach Anspruch 12, **dadurch gekennzeichnet**, daß die Senfsamen Sinapis-alba-Samen sind.

19. Biocides Mittel nach Anspruch 12, **dadurch gekennzeichnet**, daß die Senfsamen Brassica-juncea-Samen sind.

20. Biocides Mittel nach einem der Ansprüchen 12 bis 19, **dadurch gekennzeichnet**, daß das botanische Extrakt ein Glucosid ist.

21. Biocides Mittel nach einem der Ansprüche 12 bis 19, **dadurch gekennzeichnet**, daß das botanische Extrakt ein Glucosinolat ist.

22. Biocides Mittel nach einem der Ansprüche 12 bis 19, **dadurch gekennzeichnet**, daß das botanische Extrakt ein Isothiocyanat ist.

23. Biocides Mittel nach einem der Ansprüche 12 bis 19, **dadurch gekennzeichnet**, daß das botanische Extrakt Sinigrin ist.

24. Biocides Mittel nach einem der Ansprüche 12 bis 19, **dadurch gekennzeichnet**, daß das botanische Extrakt Sinalbin ist.

25. Biocides Mittel nach einem der Ansprüche 12 bis 19, **dadurch gekennzeichnet**, daß das botanische Extrakt Sinapin ist.

26. Biocides Mittel nach einem der Ansprüche 12 bis 19, **dadurch gekennzeichnet**, daß das botanische Extrakt Gluconapin ist.

27. Biocides Mittel nach einem der Ansprüche 12 bis 19, **dadurch gekennzeichnet**, daß das botanische Extrakt Glucobrassicanapin ist.

28. Biocides Mittel nach einem der Ansprüche 12 bis 19, **dadurch gekennzeichnet**, daß das botanische Extrakt Progoitrin ist.

29. Biocides Mittel nach einem der Ansprüche 12 bis 19, **dadurch gekennzeichnet**, daß das botanische Extrakt Glucotropaeolin ist.

30. Biocides Mittel nach einem der Ansprüche 12 bis 19, **dadurch gekennzeichnet**, daß das botanische Extrakt Gluconasturtin ist.

31. Biocides Mittel nach einem der Ansprüche 12 bis 19, **dadurch gekennzeichnet**, daß das botanische Extrakt Glucobrassicin ist.

32. Biocides Mittel nach Anspruch 31, **dadurch gekennzeichnet**, daß das genannte Glucobrassicin ein vier-Hydroxiglucobrassicin ist.

33. Biocides Mittel nach einem der Ansprüche 12 bis 32, **dadurch gekennzeichnet**, daß das Extrakt eine beliebige Kombination der Ansprüche 20 bis 32 umfaßt.

## Revendications

1. Agent biocide comprenant un mélange d'une substance alcaline et de graines de moutarde.

2. Agent biocide selon la revendication 1, comprenant en outre un agent édulcorant.

3. Agent biocide selon la revendication 2, dans lequel l'agent édulcorant est du miel.

4. Agent biocide selon la revendication 1, dans lequel la substance alcaline est du carbonate de bismuth.

5. Agent biocide selon la revendication 4, dans lequel le carbonate de bismuth est de qualité pure.

6. Agent biocide selon les revendications 2 à 5, dans lequel les trois composants sont mélangés selon les ratios suivants : une cuillère à café de substance alcaline, deux cuillères à soupe de graines de moutarde et une cuillère à soupe de miel.

7. Agent biocide selon la revendication 6, dans lequel lesdits composants sont pulvérisés ensemble afin d'assurer leur mélange intime.

8. Agent biocide selon la revendication 1, dans lequel les graines de moutarde sont des Sinapis-alba.

9. Agent biocide selon la revendication 1, dans lequel les graines de moutarde sont des Brassica juncea.

10. Agent biocide selon la revendication 1, dans lequel les graines de moutarde sont constituées d'un mélange de Sinapis-alba ou de Brassica juncea.

11. Agent biocide selon la revendication 10, dans lequel des quantités égales des deux dites graines sont prévues.

12. Agent biocide comprenant un mélange de substance alcaline, un extrait botanique produit à partir de la famille botanique de plantes communément connues en tant que plantes productrices de graines de moutarde, et une substance édulcorante.

13. Agent biocide selon la revendication 12, dans lequel l'agent édulcorant est du miel.

14. Agent biocide selon la revendication 12, dans lequel la substance alcaline est du carbonate de bismuth.

15. Agent biocide selon la revendication 14, dans lequel le carbonate de bismuth est de qualité pure.

16. Agent biocide selon la revendication 12, dans lequel les trois composants sont mélangés selon es ratios suivants : une cuillère à café de substance alcaline, deux cuillères à soupe de graines de moutarde et une cuillère à soupe de miel.

17. Agent biocide selon la revendication 12, dans lequel lesdits composants sont pulvérisés ensemble afin d'assurer leur mélange intime.

18. Agent biocide selon la revendication 12, dans lequel les graines de moutarde sont des Sinapis-alba.

19. Agent biocide selon la revendication 12, dans lequel les graines de moutarde sont des Brassica juncea.

20. Agent biocide selon les revendications 12 à 19, dans lequel ledit extrait botanique est un glucoside.

21. Agent biocide selon les revendications 12 à 19, dans lequel ledit extrait botanique est un glucosinolate.

22. Agent biocide selon les revendications 12 à 19, dans lequel ledit extrait botanique est un isothiocyanate.

23. Agent biocide selon les revendications 12 à 19, dans lequel ledit extrait botanique est de la sinigrine.

24. Agent biocide selon les revendications 12 à 19, dans lequel ledit extrait botanique est de la sinalbine.

25. Agent biocide selon les revendications 12 à 19, dans lequel ledit extrait botanique est de la sinapine.

26. Agent biocide selon les revendications 12 à 19, dans lequel ledit extrait botanique est du gluconapane.

27. Agent biocide selon les revendications 12 à 19, dans lequel ledit extrait botanique est du glucobrassicanipane.

28. Agent biocide selon les revendications 12 à 19, dans lequel ledit extrait botanique est de la progoitrine.

29. Agent biocide selon les revendications 12 à 19, dans lequel ledit extrait botanique est du glucotropaeoline.

30. Agent biocide selon les revendications 12 à 19, dans lequel ledit extrait botanique est du gluconasturtine.

31. Agent biocide selon les revendications 12 à 19, dans lequel ledit extrait botanique est du glucobrassicine.

32. Agent biocide selon la revendication 31, dans lequel ledit glucobrassicine est de la quatre-hydroxiglucobrassicine.

33. Agent biocide selon les revendications 12 à 32, dans lequel ledit extrait comprend l'une des combinaisons relatives aux revendications 20 à 32.
